# EUROPEAN PATENT SPECIFICATION

(11) **EP 3 164 139 B1**
(45) Date of publication and mention of the grant of the patent: **12.02.2020**
(21) Application number: 15814580.5
(22) Date of filing: 26.06.2015
(51) Int. Cl.: A61K 31/74, A61P 17/00

(54) **TOPICAL COMPOSITIONS AND METHODS FOR TREATING WOUNDS**
TOPISCHE ZUSAMMENSETZUNGEN UND VERFAHREN ZUR WUNDBEHANDLUNG
COMPOSITIONS TOPIQUES ET MÉTHODES DE TRAITEMENT DE PLAIES

(30) Priority: 01.07.2014 US 201462019644 P
(43) Date of publication of application: 10.05.2017
(73) Proprietor: Chacon, Enrique, El Paso, TX 79932 (US); Saenz, Xavier J., El Paso, TX 79922 (US)
(72) Inventor: Chacon, Enrique, El Paso, TX 79932 (US); Saenz, Xavier J., El Paso, TX 79922 (US)
(74) Representative: Wynne-Jones IP Limited
(86) International application number: PCT/US2015/037977
(87) International publication number: WO 2016/003798

(56) References cited:
- EP-A1- 2 070 531
- US-A1- 2003 232 086
- US-A1- 2004 076 671
- US-A1- 2008 206 161
- US-A1- 2008 286 299
- US-A1- 2013 245 575
- SOLIS F F ET AL: "Effectiveness of silver sulfadiazine in reepithelization of hot liquid burns affecting neutral zones in children", REVISTA CHILENA DE PEDIATRIA 200712 CL, vol. 78, no. 6, December 2007 (2007-12), pages 607-614, XP002768096, ISSN: 0370-4106

## Description

### BACKGROUND OF THE INVENTION

Skin wounds are classified into two main categories: acute and chronic. Typically, most acute wounds heal in an organized and timely fashion; however, many wounds cease to heal and continue down a path leading to cell death resulting in tissue necrosis further compromising the healing process and leading to a chronic wound. Progression to a chronic wound is often the result of an underlying medical condition such as diabetes a peripheral vascular disease or a bed-ridden or immobile condition. The longer a wound takes to heal, the greater the chances that wound progression will result in complications such as local and systemic infections, further compromising a patient's quality of life and potentially leading to amputation and death.

Chronic wounds affect an individual's quality of life and are a leading cause of burgeoning healthcare costs. Examples of the financial burden of chronic wounds on health care are clearly evident in evaluating the clinical cases and related costs associated with diabetic wounds and pressure ulcers alone. There are an estimated 26 million diabetics in the United States and 285 million globally. About 15% of diabetics will develop diabetic foot ulcers of which 50% of these will become infected resulting in approximately 82,000 amputations in the U.S. and 898,000 amputations worldwide each year. An estimated $5 - 7 billion annually is spent on diabetic foot care in the United States. An estimated $11 billion annually is spent in the U.S. on hospitalizations resulting from pressure ulcers. The yearly cost of an individual patient's pressure ulcer care typically ranges between $21,000.00 and $152,000.00. Medicare estimates that pressure ulcers add approximately $43,180.00 in cost to a patient's hospital stay.

Current medical treatments for wounds are evolving rapidly around new technologies in antimicrobials, debridement pharmaceuticals, and dressings. Most of these technologies, however, target only a single mode of action, such as the use of an antimicrobial, or a dressing that provides a hydration approach, or a debridement agent to assist in the removal of dead tissue. When using such agents, healing is extremely limited, often leading to a stasis, or inhibition of a chronic wound's progression, without effectively treating the wound.

Thus, there is a substantial and current need for improved topical pharmaceutical compositions and treatment methods that target multiple biochemical pathways/modes of action, in both the patient with the wound and the microbe(s) infecting the wound. Such improved treatments can effectively reduce the amount of time required for a wound to heal, thereby reducing medical costs and improving the quality of life of the patient.

Background art includes:
- EP 2 070 531 A1;
- US 2013/245575 A1;
- US 2004/076671 A1; and
- SOLIS F F ET AL: "Effectiveness of silver sulfadiazine in reepithelization of hot liquid burns affecting neutral zones in children", REVISTA CHILENA DE PEDIATRIA 200712 CL, vol. 78, no. 6, December 2007 (2007-12), pages 607-614, ISSN: 0370-4106

### SUMMARY OF THE INVENTION

The present invention is set out in the appended claims. Any embodiments, aspects or examples of the present description/disclosure that do not fall within the scope of said claims are provided for illustrative purposes only and do not form part of the present invention.

The present invention relates to compositions comprising agents/ingredients to treat abnormal skin conditions such as those found in wounds, and for example, in an infected wound in a subject. The subject can be a mammal, for example a dog, cat, horse, cow, pig, goat, etc. and the treatment can be in a veterinary setting. In particular, the compositions of the present invention can be used to treat a human subject. The agents of the compositions have activities that affect different biochemical reaction pathways, not only within the microbes/fungi being targeted within and surrounding the wound, but also within the host fighting the infection, thus resulting in different and multiple pharmacological responses acting in concert to heal wounds. More specifically, the present invention relates to compositions composed of various active agents that have different pharmacological properties that target the treatment of abnormal topical skin conditions at different biochemical pathways by not only inhibiting and/or eliminating microbial contamination, but also enhancing the host's ability to recover and regenerate wound damaged cellular tissue using topically active pharmaceutical agents: antimicrobial compounds, wherein the antimicrobial compounds include silver sulfadiazine, bacitracin, neomycin sulfate, and polymixin B sulphate; trace elements; tissue reconstruction supporting elements; and an analgesic wherein the agents are dispersed in a suitable carrier, such as a semi-soft or cream-like, gel- water-dispersible or water-soluble oil-in-water or water-in-oil emulsion carriers. For example, one embodiment of the present invention contains four antibiotics that have all been proven to be safe and effective against a variety of organisms. In another embodiment, the silver sulfa-containing antibiotic can be replaced with ionic silver to make an over-the-counter (OTC) formulation or for sulfa allergic individuals. In addition, the composition incorporates a form of zinc and silver known to boost the antibacterial properties of individual antibiotics. Moreover, the present invention additionally incorporates a combination of one, or more trace elements, cofactors and vitamins, e.g., Zn, Vitamin E, CoQ10, to not only boost the mammalian host's immune response to fight infections, but also to promote tissue regeneration by helping to maintain mitochondrial integrity and increase the expression of the connective tissue growth factor (CTGF) responsible for extracellular tissue wound repair. Optionally, the compositions of the present invention can incorporate a commonly used topical anesthetic to provide additional patient comfort during the tissue healing process.

The present invention relates, in one embodiment, to a topical pharmaceutical composition for treating a skin condition, comprising a combination of agents, wherein the combination includes: a) one or more antimicrobial compounds; b) one or more trace elements; c) one or more tissue reconstruction elements; and d) one or more analgesics, and a pharmaceutically-acceptable carrier. In another embodiment, the invention relates to a topical pharmaceutical composition for treating a skin condition, comprising silver sulfadiazine, zinc oxide, lidocaine, bacitracin, neomycin (e.g., neomycin sulfate), polymixin B (e.g., polymixin B sulfate), vitamin E and Coenzyme Q₁₀.

In another embodiment, the invention relates to an OTC sulfa-free topical pharmaceutical composition for treating skin wound conditions comprising ionic silver (in replacement of the silver- containing sulfa drug), zinc oxide, lidocaine, bacitracin, neomycin (e.g., neomycin sulfate) polymixin B (e.g., polymixin B sulfate), vitamin E and Coenzyme Q₁₀.

In an additional embodiment, the invention further relates to a method of treating a skin condition in a subject in need thereof, comprising administering to the subject a therapeutically-effective amount of topical pharmaceutical composition, wherein the composition comprises a combination of agents, wherein the combination includes: a) one or more antimicrobial compounds; b) one or more trace elements; c) one or more tissue reconstruction elements; and d) one or more analgesics, and a pharmaceutically-acceptable carrier. In a particular embodiment, the method comprises administering a composition comprising silver sulfadiazine, zinc oxide, lidocaine, bacitracin, neomycin (e.g., neomycin sulfate), polymixin B (e.g., polymixin B sulfate), vitamin E and Coenzyme Q₁₀.

The topical pharmaceutical compositions and methods described herein, also referred to interchangeably as pharmaceutical compositions and methods of the invention, provide improved efficacy for the topical treatment of skin conditions, such as acute and chronic wounds, compared to commercially available prescription and over-the-counter therapeutic agents and compositions that are in current use.

### BRIEF DESCRIPTION OF THE DRAWINGS

FIG. 1 is a picture of a foot of a diabetic male patient suffering from foot ulcers, who had been administered vancomycin to treat a MRSA infection.
FIG. 2 is a subsequent picture of the foot shown in FIG. 1, taken after 8 weeks of treatment with a topical pharmaceutical formulation containing silver sulfadiazine, zinc oxide, lidocaine, bacitracin, neomycin, polymixin B, vitamin E and Coenzyme Q₁₀.
FIG. 3 is a picture of a chronic pressure foot ulcer near the heel of the foot shown in FIG. 1, which was taken on the same day as the picture of FIG. 1.
FIG. 4 is a subsequent picture of the foot ulcer shown in FIG. 3, taken after 8 weeks of treatment with a topical pharmaceutical formulation containing silver sulfadiazine, zinc oxide, lidocaine, bacitracin, neomycin, polymixin B, vitamin E and Coenzyme Q₁₀.
FIG. 5A is a picture of a foot of a 51-year old female patient suffering from chronic tinea pedis.
FIG. 5B is a subsequent picture of the foot shown in FIG. 5A, taken about 2 weeks later, following a two-week treatment with a topical pharmaceutical formulation containing silver sulfadiazine, zinc oxide, lidocaine, bacitracin, neomycin, polymixin B, vitamin E and Coenzyme Q₁₀.
FIG. 6A is a photo of an almost healed large tattoo 1 day after the tattoo procedure treated with a topical formulation containing silver sulfadiazine, zinc oxide, lidocaine, bacitracin, neomycin, polymixin B, vitamin E and Coenzyme Q₁₀.
6B is a photo of a typical non-healed red and swollen large tattoo 1 day after the tattoo procedure without any topical treatment.
FIG. 7 is a picture of microbes growing on a culture plate. Each of the 5 sections of the plate contains three 6 mm paper disks, each containing one or more test agent(s). The test agents are represented by the following nomenclature: O represents oxacillin (a β-lactam penicillin); V represents vancomycin; X represents a topical pharmaceutical formulation containing silver sulfadiazine, zinc oxide, lidocaine, bacitracin, neomycin, polymixin B, vitamin E and Coenzyme Q₁₀; N represents Neosporin®; and S represents Silvadene®.

### DETAILED DESCRIPTION OF THE INVENTION

A description of example embodiments of the invention follows.

Unless defined otherwise, all technical and scientific terms used herein have the same meaning as commonly understood by one of ordinary skill in the art to which this invention pertains.

The present invention is based, in part, upon the identification of a therapeutically-effective topical pharmaceutical composition comprising a combination of antimicrobials, trace elements, tissue reconstruction supporting elements, and an analgesic. This combination has been shown to facilitate regenerative skin tissue repair, particularly in the treatment of wounds, by enhancing the efficiency of skin tissue repair and by providing antimicrobial properties.

Thus, the invention relates, in part, to pharmaceutical compositions for regenerative medicine and their uses. The term "regenerative medicine" is used herein to refer to a process of regenerating human cells and tissues to restore or establish normal function (e.g., the medical practice of treating a skin condition, such as a wound). Regeneration can be achieved, for example, by replacing damaged tissue and/or by stimulating the body's own repair mechanisms to heal previously irreparable tissues.

The pharmaceutical compositions of the invention comprise a combination of various active agents having different pharmacological properties that are useful for treating a variety of skin conditions. Accordingly, in one embodiment, the invention relates to a topical pharmaceutical composition for treating a skin condition, comprising a combination of agents, wherein the combination includes: a) one or more antimicrobial compounds; b) one or more trace elements; c) one or more tissue reconstruction elements; and d) one or more analgesics, and a pharmaceutically-acceptable carrier.

Numerous antimicrobial compounds are known in the art. Representative classes of antimicrobial compounds that are suitable for use in the compositions described herein include, but are not limited to, sulfonamides (e.g., ionic silver, silver sulfadiazine, sulfacetamide, sulfamethoxazole, sulfasalazine, sulfamethizole); aminoglycosides (e.g., neomycin, tobramycin, gentamycin, amikacin, kanamycin, spectinomycin, paromomycin, netilmicin); cyclic polypetides (e.g., bacitracin, polymixin B); antifungals (e.g., nystatin, terbinafine, butenafine, ketoconazole, itraconazole, tolnaftate, crystal violet, benzoic acid); beta-lactam antibiotics; cephalosporins; carbapenems (e.g., penicillin, ampicillin, amoxicillin, methicillin, cefazolin, cefaclor, ceftriaxone, cefepime, ceftobripole, imipenin, ertapenem); quinolones; oxazolidinones (e.g., ciprofloxacin, levofloxacin, ofloxacin); macrolides (e.g., erythromycin, azithromycin, clarithromycin); tetracyclines (e.g., tetracycline, doxycycline, minocycline); glycopeptides (e.g., vancomycin, telavacin); t-RNA synthetase inhibitors (e.g., mupirocin); lipopeptides (e.g., daptomycin); lincosamides (e.g., clindamycin, lincomycin); nitrofurantoins (e.g., nitrofurantoin, furazolidone); metronidazole; chloramphenicol; and tinidazole.

The topical pharmaceutical composition of the invention includes each of silver sulfadiazine, bacitracin, neomycin (e.g., neomycin sulfate, and polymixin B (e.g., polymixin B sulfate).

In a particular embodiment, the topical pharmaceutical composition of the invention includes about 0.1 to about 5% by weight of silver sulfadiazine. Preferably, the composition includes about 0.25% by weight of silver sulfadiazine or ionic silver at 5-200 ppm, e.g., at a concentration of 50 ppm. In another embodiment, the topical pharmaceutical composition of the invention includes about 0.1 to about 4000 Units per gram bacitracin. Preferably, the composition includes about 400 Units per gram bacitracin.

The topical pharmaceutical composition of the invention further includes one or more trace elements. "Trace element" refers to both agents that act to enhance antimicrobial effectiveness (e.g., zinc, silver, copper, CoQ10) and agents that act to promote the immune response (e.g., zinc, selenium, copper, iron, vitamin C, vitamin E, CoQ10. In one embodiment, the one or more trace elements are selected from the group consisting of zinc, silver, selenium, copper, iron, magnesium, vitamin C, vitamin E and Coenzyme Q₁₀, or a combination thereof. Preferably, the topical pharmaceutical composition of the invention includes zinc (e.g., zinc oxide) as trace element.

In one embodiment, the topical pharmaceutical composition of the invention includes about 0.1 to about 40% by weight of zinc. Preferably, the composition includes about 5% by weight of zinc (e.g., about 5% by weight of zinc oxide). Zinc oxide is commonly used in ointments, creams, and lotions as a sunscreen to protect the skin from ultraviolet. In addition to being used in sun screens, zinc is commonly used in "common cold" preparations to boost the immune system. Zinc ion is a trace element that is essential for cells of the immune system. Zinc deficiency affects the ability of immune cells to function properly. Other trace elemental metals and substrates that have been shown to promote the immune response are selenium, copper, iron, vitamin C, vitamin E, CoQ10. Zinc oxide has also been shown to boost the effectiveness of the antibacterial properties of other antibiotics such as Ciprofloxacin against staphylococcus aureus and Escherichia coli.

In addition, the topical pharmaceutical composition of the invention includes one or more tissue reconstruction elements. "Tissue reconstruction support elements" and "tissue reconstruction elements" are used interchangeably to refer to agents that support tissue regenerative biochemical reactions used in cell division (e.g., biochemical reactions in mitochondrial function integrity and cell replication pathways), such as those seen in tissue reconstruction. In certain embodiments, the one or more tissue reconstruction elements can be selected from the group consisting of vitamin E, Coenzyme Q₁₀, L-carnitine, choline, folic acid, magnesium , oleic acid, caprylic acid, linoleic acid, lauric acid and taurine, or a combination thereof. In a particular embodiment, the one or more tissue reconstruction elements include both vitamin E and Coenzyme Q₁₀.

In one embodiment, the topical pharmaceutical composition of the invention includes about 0.05 to about 120,000 Units per gram vitamin E. Preferably, the composition includes about 350 Units per gram vitamin E. Vitamin E has a variety of pharmacological functions. Its most common function being recognized as an antioxidant. A more important discovery of the biological functions of vitamin E acting as an antioxidant is its role in acting in cell signaling. Vitamin E also has an effect on gene expression by modulating the expression of the connective tissue growth factor (CTGF) responsible for extracellular tissue wound.

In another embodiment, the topical pharmaceutical composition of the invention includes about 1 to about 100 mg per gram Coenzyme Q₁₀. Preferably, the composition includes about 15 mg per gram Coenzyme Q₁₀. Coenzyme Q₁₀, also known as ubiquinone-10, coenzyme Q, and abbreviated at times to CoQ10. This oil-soluble, vitamin-like substance is known more for its antioxidant properties than for its main role in eukaryotic cells. CoQ10 is found primarily in the mitochondria. It is an essential component of the mitochondrial electron transport chain and participates in aerobic cellular respiration, to facilitate energy production in the form of ATP. Mitochondrial integrity is of utmost importance in cell survival and replication. Cell death is caused by a variety of biochemical malfunctions of which apoptosis is a preprogrammed cell death that begins a cascade of events initiated at the mitochondrial level. Apoptosis is triggered by the opening of a protein complex pore on the mitochondrial membrane surface that upon opening uncouples the electron flow down the mitochondrial respiratory chain resulting in a cessation of ATP production, a disruption in calcium homeostasis, and an increased electron leakage that produces reactive oxygen species all causing lethal perturbations in a cells ability to maintain it normal physiological functions. Coenzyme Q₁₀ is an integral part of the mitochondrial respiratory chain along with other substrates and co factors such as fatty acid oxidation, magnesium, calcium, carnitine, choline, taurine, and folic acid. The present invention not only incorporates ubiquinone to support mitochondrial respiration but also Vitamin E acting to further modulate connective tissue growth factor and as antioxidants to reduce mitochondrial reactive oxygen species.

The topical pharmaceutical composition of the invention can further include one or more analgesics. Suitable analgesics for inclusion in the topical pharmaceutical compositions are well known in the art. In a particular embodiment, the one or more analgesics the topical pharmaceutical compositions of the invention are derived from cocaine. Preferred analgesics for inclusion in the pharmaceutical compositions described herein include, but are not limited to, lidocaine, pramoxine, benzocaine, tetracaine, dibucaine, butamben, oxybuprocaine, proparacine, and proxymetacaine, or a combination thereof.

In a particular embodiment, the pharmaceutical compositions described herein include lidocaine. In one embodiment, the topical pharmaceutical composition of the invention includes about 0.1 to about 25% by weight of lidocaine. Preferably, the composition comprises about 0.5% by weight of lidocaine.

In another embodiment, the invention relates to a topical pharmaceutical composition for treating a skin condition, comprising silver sulfadiazine, zinc oxide, lidocaine, bacitracin, neomycin (e.g., neomycin sulfate), polymixin B (e.g., polymixin B sulfate), vitamin E and Coenzyme Q₁₀. Typical concentration ranges for silver sulfadiazine, zinc oxide, lidocaine, bacitracin, neomycin (e.g., neomycin sulfate), polymixin B (e.g., polymixin B sulfate), vitamin E and Coenzyme Q₁₀ in a topical pharmaceutical composition of the invention are:

| | |
|---|---|
| • Silver sulfadiazine | about 0.1 to about 5% |
| • Zinc oxide | about 1 to about 40% |
| • Lidocaine | about 0.1 to about 25% |
| • Bacitracin | about 0.1 to about 4000 Units per gram |
| • Neomycin Sulfate equivalent to | about 0.5 to about 500 mg per gram of neomycin base |
| • Polymixin B | about 50 to about 50,000 Units per gram as polymixin B sulfate |
| • Vitamin E | about 0.05 to about 120,000 Units per gram |
| • Coenzyme Q₁₀ | about 1.5 to about 1500 mg per gram |

In one embodiment, a topical pharmaceutical composition of the invention can be prepared using the following exemplary ingredients:

| | |
|---|---|
| • 1% Silver sulfadiazine ointment | 25 parts to 100 parts final equivalent to 0.25% |
| • 20% Zinc oxide cream/ointment | 25 parts to 100 parts final equivalent to 5% |
| • 2% Lidocaine cream/ointment/jelly | 25 parts to 100 parts final equivalent to 0.5% |
| | |
| • Triple antibiotic cream/ointment | 15 parts to 100 parts final equivalent to 7.5% |
| • Vitamin E Oil 70,000 U/ml | 5 parts to 100 parts equivalent to 3500 U/gm |
| • Coenzyme Q₁₀ 1500 mg | 5 parts to 100 parts equivalent to 1500 mg |

In a particular embodiment, the invention relates to a topical pharmaceutical composition for treating a skin condition, comprising the following active ingredients at the specified concentrations:

| | |
|---|---|
| • Silver sulfadiazine | about 0.25 % |
| • Zinc oxide | about 5 % |
| • Lidocaine | about 0.5 % |
| • Bacitracin | about 400 Units per gram |
| • Neomycin Sulfate equivalent to | about 3.5 mg per gram of neomycin base |
| • Polymixin B | about 5000 Units per gram as polymixin B sulfate |
| • Vitamin E | about 350 Units per gram |
| • Coenzyme Q₁₀ | about 15 mg per gram |

In another embodiment, the invention relates to a topical pharmaceutical composition for treating a skin condition, the composition comprising ionic silver, zinc oxide, lidocaine, bacitracin, neomycin (e.g., neomycin sulfate) polymixin B (e.g.,, polymixin B sulfate) vitamin E and Coenzyme Q₁₀. In this embodiment the compositon can be prepared using the following exemplary ingredients:

| | |
|---|---|
| • Ionic silver | about 5 to about 200 ppm |
| • Zinc oxide | about 1 to about 40% |
| • Lidocaine | about 0.1 to about 2.5% |
| • Bacitracin | about 0.1 to about 4000 Units per gram |
| • Neomycin Sulfate base equivalent to | about 0.5 to about 500 mg per gram of neomycin |
| • Polymixin B | about 50 to about 50,000 Units per gram as polymixin B sulfate |
| • Vitamin E | about 0.05 to about 120,000 Units per gram |
| • Coenzyme Q₁₀ | about 1.5 to about 1500 mg per gram |

In various embodiments, the topical pharmaceutical compositions of the invention can include one or more pharmaceutically-acceptable carriers, excipients and/or additives as inactive ingredients. Suitable pharmaceutically-acceptable carriers, excipients and/or additives for inclusion in the pharmaceutical compositions of the invention are well known in the art and include, for example, water, buffered water, normal saline, 0.4% saline, 0.3% glycine, hyaluronic acid, stabilizers, antioxidants, osmolality adjusting agents, buffers, and pH adjusting agents, pH stabilizing agents, humectants, emollients, preservatives, chelating agents (e.g., DTPA or DTPA-bisamide, calcium DTPA, CaNaDTPA-bisamide), antioxidants, physiologically biocompatible buffers (e.g., tromethamine hydrochloride), or, optionally, additions of calcium or sodium salts (e.g., calcium chloride, calcium ascorbate, calcium gluconate or calcium lactate). Preferably, the pharmaceutically-acceptable carrier is a dermatologically-acceptable carrier.

Exemplary inactive ingredients that are suitable for inclusion in the pharmaceutical compositions of the invention include, for example, petrolatum, one or more long chain fatty alcohols (e.g., a fatty alcohol having a carbon atom content in the range C₁₆ - C₂₂), a non-ionic emulsifying agent, a humectant (e.g., propylene glycol, sorbitol, glycerin), an emollient, a preservative (e.g., methylparaben, propylparaben), a cellulose gum derivative (e.g., carboxymethylcellulose) and water, or a combination thereof.

In a particular embodiment, the topical pharmaceutical composition of the invention comprises about 0.1 to about 5% by weight of silver sulfadiazine, about 0.1 to about 4000 Units per gram bacitracin; neomycin sulfate; polymixin B sulfate, about 0.1 to about 40% by weight of zinc oxide, about 0.05 to about 120,000 Units per gram Vitamin E; Coenzyme Q₁₀, about 0.1 to about 25% by weight of lidocaine, about 0-25 percent by weight petrolatum, about 7-45 percent by weight of a long chain fatty alcohol having a carbon atom content in the range C₁₆-C₂₂, about 4-16 percent by weight of a non-ionic emulsifying agent, about 7-40 percent by weight of a humectant (e.g., a humectant selected from the group consisting of propylene glycol, sorbitol and glycerin), 0-15 percent by weight of an emollient and an amount of water, and, optionally, preservatives (e.g., methylparaben, propylparaben), cellulose gum derivatives (e.g., carboxymethylcellulose sodium), sufficient to bring the total of the percentages of the above-identified components to 100 percent.

Also, in some embodiments, the pharmaceutical compositions of the invention may contain, if desired, other compatible therapeutic agents for treating skin conditions.

The pharmaceutical compositions of the invention are intended for topical administration. Accordingly, the compositions described herein can be prepared in suitable forms to be applied to the skin, or mucus membranes of the nose and throat, and may take the form of ointments, creams, gels (e.g., hydrogels), emulsions, poultices, lotions, foams, suspensions, pastes, tinctures, or a liquid dosage form (e.g., topical solution, emulsion, suspension), or even solid stick. Alternative topical formulations include shampoo preparations, oral pastes and mouthwash. Such topical compositions can further include chemical compounds such as dimethylsulfoxide (DMSO) to facilitate penetration of the active ingredient through the surface of the skin.

In one embodiment, the topical pharmaceutical compositions described herein are formulated as a liquid dosage form, preferably as oil-in-water emulsion using, for example, mineral oils, petrolatum and the like. In a particular embodiment, the topical pharmaceutical composition is prepared as an oil-in-water emulsion with a water-soluble carrier or water-dispersible carrier. In a further embodiment, the oil-in-water emulsion has a cream-like or semi-soft texture.

The pharmaceutical compositions of the invention can be provided in unit dosage form. In a particular embodiment, the composition is in a dosage form suitable for application to skin. For example, the composition can be subdivided into unit doses containing appropriate quantities of the active ingredient(s). The unit dosage form can be a packaged preparation, the package containing discrete quantities of, for example, ointment, lotion, or cream in packets, tubes, jars, vials or ampules.

### Methods of Treating Skin Conditions

The topical pharmaceutical compositions of the invention are useful for treating a variety of skin conditions. Accordingly, in an additional embodiment, the invention further relates to the claimed composition for use in a method of treating a skin condition in a subject in need thereof, comprising administering to the subject a therapeutically-effective amount of topical pharmaceutical composition, wherein the composition comprises a combination of agents, wherein the combination includes: a) silver sulfadiazine, bacitracin, neomycin sulfate, and polymixin B sulfate; b) one or more trace elements; c) one or more tissue reconstruction elements; and d) one or more analgesics, and a pharmaceutically-acceptable carrier.

As used herein, "skin condition" refers to any disorder, syndrome, anomaly, pathology, or abnormal characteristic of the skin or underlying connective tissue. Exemplary skin conditions include, but are not limited to, acute and chronic wounds, burns (e.g., caustic burns, sun burns), rashes, various forms of acne vulgaris and related conditions, such as, *e.g.,* seborrhea (scaly red skin), comedones (whiteheads and blackheads), papules (pinheads), nodules (large pimples), pimples, scaring, cellulitis, chaffing and blisters (e.g., from athletic or labor-intensive physical activities), post-surgical wounds and herpes skin sores.

In one embodiment, the skin condition is a wound. The wound to be treated can be an open wound or a closed wound and, furthermore, can be acute or chronic and, additionally, can be healing or non-healing. The most significant and thus problematic wounds are chronic wounds. Chronic wounds are wounds that appear to be stagnated in the inflammatory or proliferative phase. Chronic wounds can be infected or non-infected with microbial growth. Their degree of damage is rated by stages. These stages reflect the intensity of the wound damage and range from Stage 1 to Stage 4. Stage 1 wounds are characterized by a swelling, redness, and a possible warm feel of the outer most skin layer (epidermis). Stage 2 wounds are characterized by damage and rupture of the outer epidermis tissues with some penetration into the inner dermal layers of skin. Stage 3 wounds can be characterized by visualization of the adipose fat layers such that the upper dermis and epidermis have been destroyed. Stage 4 wounds are wounds such that there is a significant destruction deep into the adipose fat layer and eventually to the extent that bone can be visualized. Exemplary wounds to be treated with the topical pharmaceutical compositions described herein include abrasions (e.g., road rash), blisters, bruises, cuts, scrapes, puncture wounds (e.g., bite wounds, stab wounds), and tattoos.

In a particular embodiment, the skin condition is a chronic wound. In a further embodiment, the chronic wound is a diabetic wound, a decubitus pressure ulcer wound, or a wound caused by a fungal infection.

The terms "treat," "treating," and "treatment" mean to counteract (e.g., reduce the probability/likelihood of developing, delay the onset of, lessen the severity of) one or more symptoms of a skin condition (e.g., wound) to the extent that the skin condition is improved according to a clinically-acceptable standard.

As used herein, the terms "subject" and "patient" refer to a mammal (e.g., human, dog, cat, horse, cow). Preferably, the subject is a human (e.g., a human who has, or is at risk for developing, a skin condition). A "subject in need thereof' refers to a subject who has, or is at risk for developing, a skin condition. A skilled medical professional (e.g., physician) can readily determine whether a patient has, or is at risk for developing, a skin condition.

An "effective amount" is an amount sufficient to achieve a desired therapeutic or prophylactic effect under the conditions of administration, such as an amount sufficient to inhibit (i.e., reduce, decrease, prevent) a skin condition (e.g., wound) in a patient who has, or is at risk for developing, a skin condition. The effectiveness of a therapy (e.g., wound healing) can be readily determined by a skilled medical professional.

The dosages of the pharmaceutical compositions described herein that are administered to a subject can vary depending upon the requirements of the particular subject, the severity of the condition being treated, and the composition being employed. Determination of the proper dosage for a particular situation is within the skill in the art.

In one embodiment, the method of treating a skin condition comprises administering a composition comprising silver sulfadiazine, zinc oxide, lidocaine, bacitracin, neomycin (e.g., neomycin sulfate), polymixin B (e.g., polymixin B sulfate), vitamin E and Coenzyme Q₁₀.

In a particular embodiment, the method of treating a skin condition comprises administering a composition comprising:

| | |
|---|---|
| • Silver sulfadiazine about | 0.25 % |
| • Zinc oxide | about 5 % |
| • Lidocaine | about 0.5 % |
| • Bacitracin | about 400 Units per gram |
| • Neomycin Sulfate equivalent to | about 3.5 mg per gram of neomycin base |
| • Polymixin B | about 5000 Units per gram as polymixin B sulfate |
| • Vitamin E | about 350 Units per gram |
| • Coenzyme Q₁₀ | about 15 mg per gram |

The pharmaceutical compositions of the invention can be administered to a subject in need thereof in any suitable topical form including, but not limited to, ointments, creams, gels, poultices, lotions, foams, suspensions, pastes or tinctures.

In addition, the pharmaceutical compositions of the invention can be administered manually or with the aid of a machine or device. In some embodiments, the pharmaceutical compositions are administered to the subject on a bandage or patch (e.g., transdermal patch). In other embodiments, the pharmaceutical compositions of the invention can be administered to the subject as a spray. For example, the topical composition can be administered using a device or method designed to more readily break the skin barrier and provide the agents in the topical formulation with a faster or more effective means through the stratum corneum. For example, this type of delivery could include, for example, use of oxygen nebulizers and nanosomal mist in conjunction with iontophoresis. A spray or nebulizer may be used to create -a nanosomal mist. In one embodiment, a micro-electronic cosmetic delivery mechanism can be used for delivery of the topical agent to the skin. This method is useful for delivering ionizable compounds to the skin and aids the penetration of small molecules through the stratum corneum.

In general, the methods for delivering the disclosed pharmaceutical compositions of the invention *in vivo* utilize art-recognized protocols for topical delivery of therapeutic compositions, with the only substantial procedural modification being the substitution of the compositions described herein for the compositions in the art-recognized protocols.

### EXEMPLIFICATION

### Example 1: Therapeutic Treatment of Chronic Wounds with a Topical Pharmaceutical Composition of the Invention

### Methods

Twenty patients with a variety of chronic wound conditions that included diabetic foot ulcers, decubitus pressure ulcers, surgical wounds, toe fungus were treated for eight weeks by wound care physicians with a compounded topical pharmaceutical composition made from compendial ingredients.

Specifically, the test pharmaceutical composition for these studies was prepared by compounding: 25 parts 1% silver sulfadiazine cream: 25 parts 20% zinc oxide ointment; 15 parts triple antibiotic ointment; 25 parts 2% lidocaine viscous; 5 parts Vitamin E Oil (as 200IU/gm final); and 5 parts Coenzyme Q₁₀ (as 15mg/gm final).

Some of the more severe cases treated were patients who had been suffering from chronic wounds for months to years. Chronic wounds spanned the entire wound rating scale from Stage 1 to Stage 4. Where Stage 4 is the most extreme condition in that the tissue has eroded beyond the epidermis, dermis, and underlying fat layers to almost exposed bone. In one of the most severe cases, one patient had already faced amputation of the toes due to a lack of wound healing and a continuous loss of tissue. In most of the severe cases, patients had been treated with a multitude of prescription drugs that are considered to be the most advanced products available. These patients were switched from their standard treatments to treatment with a topical pharmaceutical composition of the present invention because they had exhibited no improvements to their chronic wounds with the standard treatments. Three of the 20 patients were on a path headed towards amputations.

### Results

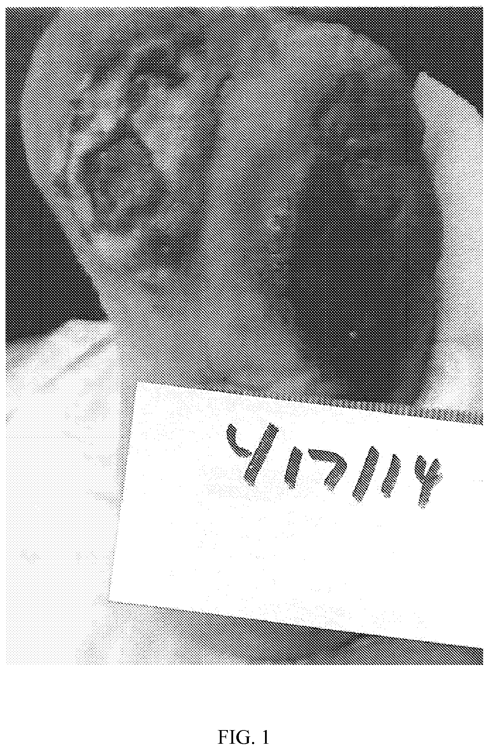

One of the twenty patients in the study had previously been treated with vancomycin in an effort to combat a MRSA infection and had already had his toes amputated due to incurable chronic diabetic foot wound infections in a continued state of decline (FIG. 1, picture taken on 01/17/2014). Note the stage 4 characteristics of both wounds on the underside of the foot. There is a dark reddish/black dead necrotic tissue due to a lack of oxygen, deterioration showing a brownish muscle and surrounding yellow/white fat layer beneath towards the bone. There is also an obvious wet and oozing appearance on the wounds. One wound begins just beneath the amputated toe region and extends beyond the length of the ball near the arch and spans more than 50% the width of the underside of the foot. The second wound on the same foot is located on the outer most side and extends from just beneath the toe amputation region along the side of the top and bottom sides along the side of the foot down past the back end of the arch to about one half of the entire arch area. The patient in FIG. 1 was treated with the test pharmaceutical composition described hereinabove and improvement was initially observed as an onset of stasis that was evident in as few as 2 to 3 days. This was the first time either of these wounds had stopped in their declining path towards cell death. Tissue deterioration had stopped.

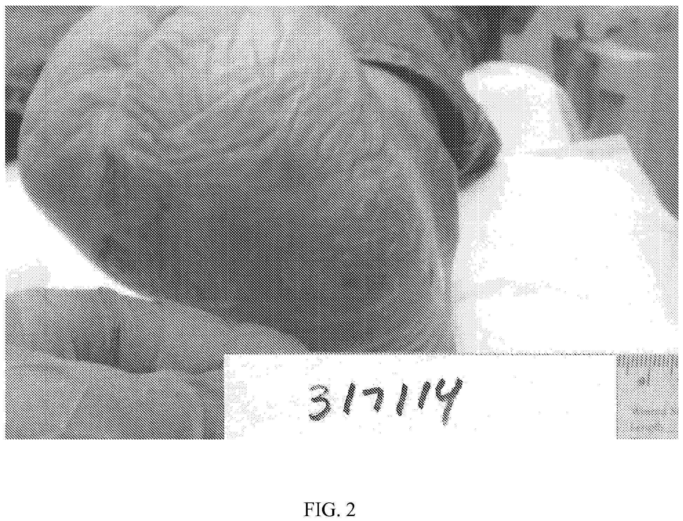

An unexpected and significant recovery towards a normal tissue was observed within 8 weeks after beginning treatment with the test pharmaceutical composition (FIG. 2, picture taken on 03/07/2014). After 8 weeks of treatment, there was a return of the normal color to the tissue to a natural pink and exhibiting distinct morphology of a healthy tissue indicative of a reestablishment of blood flow to the underlying regions. Both of the wounds unexpectedly exhibited a rapid recovery to an almost complete healing. Both wounds had returned to a stage 1 appearance in color, with signs of minimal abnormal appearance from residual scarring tissue, and with essentially minimal to no signs of open wet oozing substances, nor swollenness, nor warm to the touch. The tissue had not completely healed in the short 8 week time frame but it un-expectantly had returned to almost normal conditions as is obvious in the photographs which appear to exhibit more of a stage 1 to stage 2 wound.

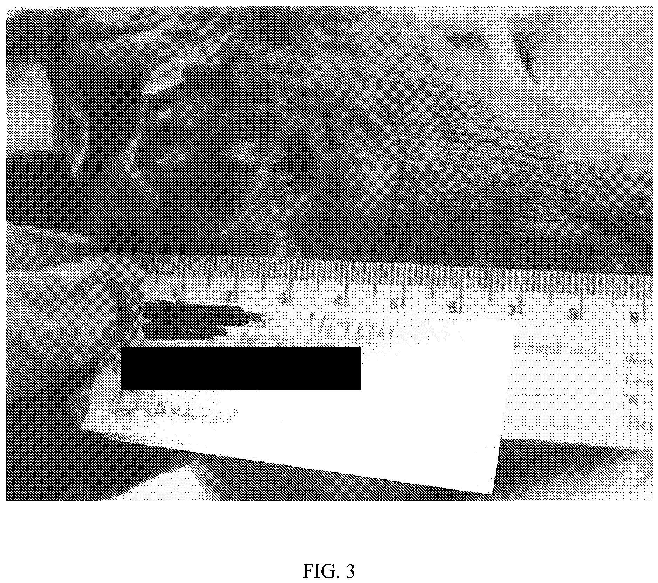

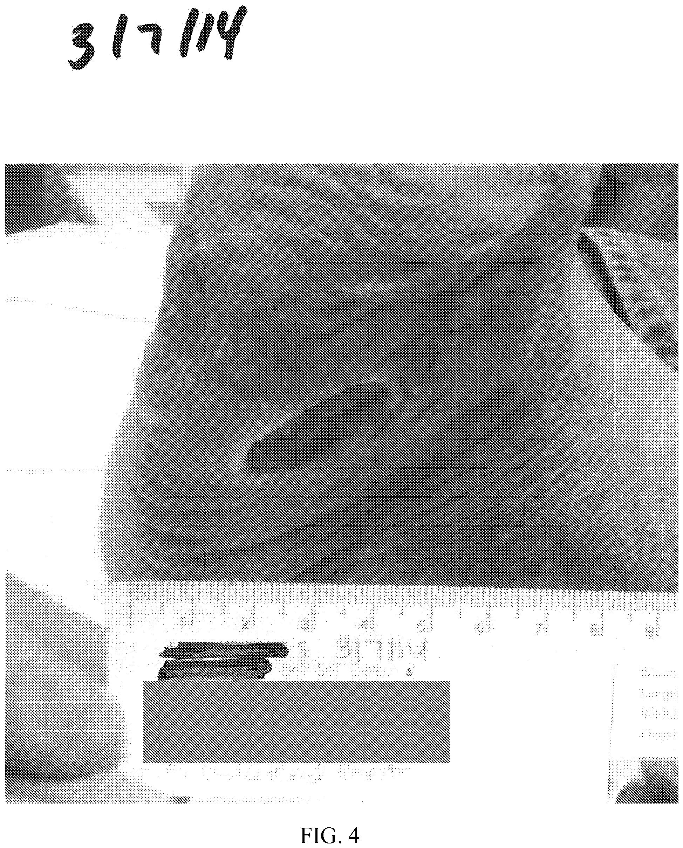

This same patient had a chronic pressure foot ulcer wound near the heel (FIG. 3, picture taken 01/17/2014). Pressure ulcers have a characteristic round/circular pattern which is not evident in the picture due to the extensive necrosis surrounding the large deep wound that spans approximately 3.5 cm wide x 5 cm high area. What is evident is the characteristic Stage 4 appearance of a total destruction of the upper and inner skin layer beyond the dermal region showing exposed yellow fatty tissue and some deep holes that extend deep into the adipose layer nearing the bone. This particular wound also exhibits a wet oozing appearance and the obvious dark reddish/black color indicative of tissue necrosis. Similarly, after only 8 weeks of treatment shown in FIG. 4, there is an obvious return towards normal looking tissue. The characteristic circular pattern of the pressure ulcer is now visible with clear and clean distinct edges. More importantly, is the rapid recovery of 8 weeks and the surprising reduction to approximately more than one-half the original wound size to ∼1 x 3 cm area from an original ∼3 x 5 cm area at the onset of treatment. The recovery is at least at stage 2 and has the appearance of more normal healthy tissue. The color has returned to a more normal pinkish tone suggesting blood flow beneath the surface. There is no evidence of the wet oozing exudate.

Three (3) of the twenty (20) patients treated had such sever chronic wounds that they had been recommended to have foot and/or partial leg amputations as the next mode of treatment. Unexpectedly, all 3 of these patients each treated for less than 8 weeks with the invention herein did not have to undergo amputation and continue to show improvement in their healing and quality of life.

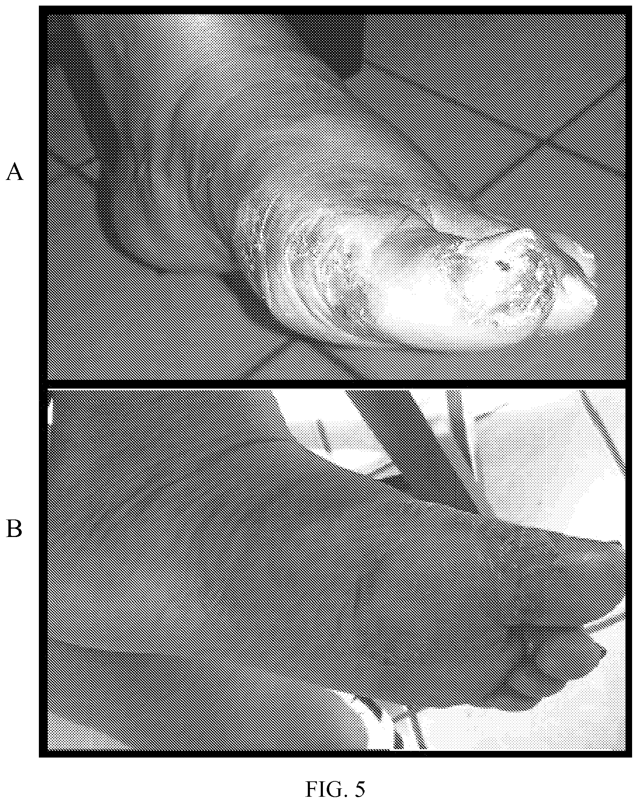

A case of tinea pedis was also reported in one of the patients. A 51-year old female who had been suffering from chronic tinea pedis infection (athlete's foot) for over 10 months had tried numerous over-the-counter remedies with no success. She was prescribed the oral antibiotic terbinafine. After a month's treatment with terbinafine, there was no sign of reduction in the infection, the patient was still complaining of a chronic itch (FIG. 5A). The terbinafine was discontinued and the patient was placed on the test pharmaceutical formulation of the invention on April 14, 2014. She returned for a follow-up with her physician about one week later on April 22, 2014. The physician's report states: "Improvement noted, fungus appears to stop, skin beginning to heal". The patient returned for a second follow up on April, 29, 2014 (FIG. 5B). The physician's report about 2 weeks after beginning treatment states: "Patient reports foot no longer itchy. Skin healing. *No signs and symptoms of infection. Foot dry, healthy new tissue visualized".* The formulation not only stopped the progression of a continued 10-month foot fungal infection, it also promoted a rapid healing response and recovery within 2 weeks.

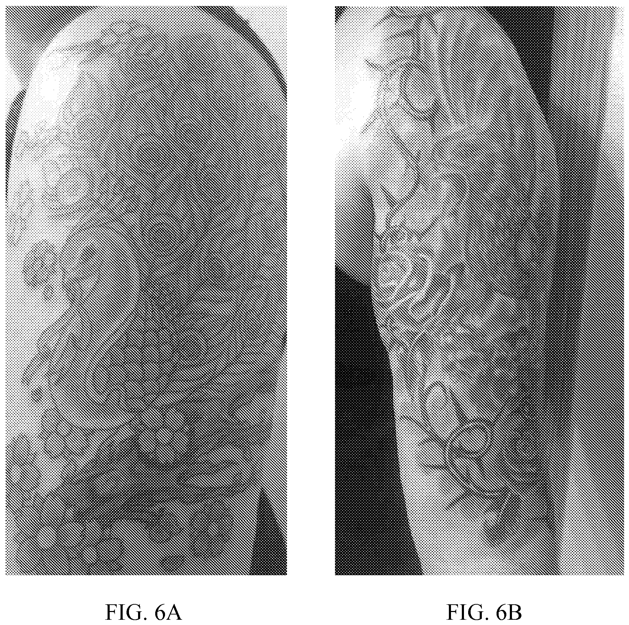

In the tattoo case presented in FIGs 6A and 6B, an impressive significant rapid wound healing was observed with not redness, swelling or oozing of the wound following the tattoo procedure when treated with the topical composition with the silver sulfadiazine composition of the present invention compared to a non-treated tattooed individual, thus allowing the treated individual to continue with subsequent tattoo sessions much sooner (2-4 days) versus the conventional about 14 days). Furthermore, over time, there was a significant and impressive preservation of the vibrant colors of the tattoo as a result of the rapid wound healing in the treated individual versus the non-treated individual.

In all cases, significant and rapid wound stasis was observed, followed by recovery due to tissue regeneration with the return of normal color of the epithelial tissue, all of which were unexpected results. The return of color to the affected tissue can be monitored to ascertain whether treatment with the test pharmaceutical composition of the invention is re-establishing blood flow into the underlying tissue.

### Example 2: Effects of a Topical Pharmaceutical Composition of the Invention on Microbial Growth

The unexpected clinical results obtained using a topical pharmaceutical composition of the invention in the study described in Example 1, including the therapeutic efficacy of the pharmaceutical composition on severe wounds, even in patients suffering from chronic MRSA infections, further prompted additional investigation of the composition's effectiveness in an *in vitro* setting.

*In vitro* culture and sensitivity studies were performed on common microbes seen in acute and stubborn chronic wound cases. The particular microbes tested included streptococcus pyogenes, staphylococcus aureus, methicillin resistant staphylococcus aureus (MRSA) and, a fungus, candida albicans. Ten different strains of MRSA bacteria obtained from infected hospital patients were tested.

The different microbes were inoculated on Mueller Hinton Agar media in culture plates. 6 mm paper disks containing different test agents - either oxacillin (a β-lactam penicillin), vancomycin, Neosporin®, Silvadene® or the pharmaceutical composition of the invention described in Example 1 (labeled "X" in FIG.-7 and Tables 1 and 2) - were placed on the culture media in triplicate in different sections of the plate. The diameter of inhibition zones of the drug treatments were measured in mm and compared to vancomycin-treated disks and methicillin-treated disk as positive and negative controls, respectively. Methicillin is a first generation β-lactam (penicillin derivative) that is typically used to treat staphylococcus. However, genetically mutated resistant strains of staphylococcus have evolved and are now the cause of numerous infections because of their resistant nature to most antibiotics. Hence, the name MRSA (methicillin resistant staphylococcus aureus). Vancomycin is a very potent yet toxic antibiotic typically used systemically to treat MRSA infections, yet vancomycin does not exhibit antifungal properties

Representative visual results of the antibiotic inhibition experiments are shown in FIG. 6-7 The agar plate shown in FIG. -7 was inoculated and grown to confluency with MRSA strain number 11, as seen by a clouded, opaque yellowish appearance. Note the clear circular area of inhibition zone surrounding the three vancomycin containing disks acting as a positive control in these experiments showing that we are killing or inhibiting the growth of MRSA #11. Conversely, note the homogeneous growth and lack of any zone inhibition surrounding the oxacillin disks, which is indicative of a methicillin resistant staphylococcus aureus (MRSA microbe).

The *in vitro* zone inhibition study design and results are summarized in Table 1. Four different microorganisms were tested. These four microbes are commonly termed Strep, for Streptococcus pyogenes; Fungi, for Candida albicans; Staph for Staphylococcus aureus; and Super Staph, for methicillin resistant staphylococcus aureus (MRSA). Treatment with oxacillin showed antimicrobial activity against S. pyogenes and the "normal" S. aureus. As anticipated, methicillin had no activity against the fungus, C. albicans, or MRSA. The incorporation of methicillin into each agar plate along with the other test drugs serves as a negative control indicator showing a methicillin-resistant microbe is present. Vancomycin shows potent activity against all the bacteria including MRSA, but is not effective against fungi. The incorporation of vancomycin into each agar test plate serves as a positive control against MRSA containing agar plates. Neosporin® exhibits inhibition activity against two bacterial strains, namely, S. pyogenes and non-resistant S. aureus (Table 1). As is to be expected, Neosporin® has no activity against MRSA or fungi. Contrarily, both Silvadene® and an exemplary pharmaceutical composition of the invention ("X") displayed activity against all of the microbes tested (Table 1).

| **TABLE 1** | | | | | | |
|---|---|---|---|---|---|---|
| | ***in vitro* Zone Inhibition Studies** | | | | | |
| **Nickname** | **Microbe** | **Oxacillin** | **Vancomycin** | **Neosporin®** | **Silvadene®** | **X** |
| **Strep** | **S. pyogenes** (n = 6) | **30.2** | **22.2** | **14.7** | **10.2** | **19.5** |
| **Fungi** | **C. albicans** (n = 6) | **0.0** | **0.0** | **0.0** | **10.7** | **10.5** |
| **Staph** | **S. aureus** (n=6) | **20.3** | **17.3** | **14.3** | **11.7** | **14.3** |
| **Super Staph** | **MRSA** (10 strains, n = 30) | **0.0** | **18.9** | **0.0** | **11.6** | **12.5** |
| Mean inhibition diameter (*mm*) | | | | | | |

Surprisingly, a mere 1 mm diameter difference observed in a zone inhibition study can indicate a significant difference. Statistics were performed to determine any significant differences observed between the drugs' ability to inhibit microbial growth within each of the microbes tested (Table 2). The only non-significant differences occurred in the fungi treated with pharmaceutical composition "X" of the invention compared to Silvadene® and in the S. aureus treated with pharmaceutical composition "X" of the invention compared to Neosporin® (Table 2). All other comparisons within each microbe tested against the different drugs were significantly different.

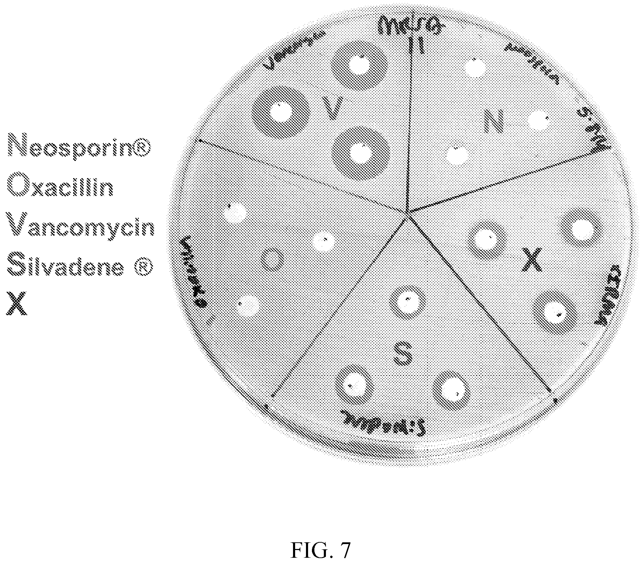

Importantly, the Neosporin® and Silvadene® tested in the zone inhibition study were from commercially available sources and contained concentrations of their active ingredients (bacitracin, neomycin and polymixin B in the case of Neosporin® and silver sulfadiazine in the case of Silvadene®) that were four (4) times more concentrated than the concentration of the corresponding compounds in pharmaceutical composition "X" of the invention (Tables 1 and 2). The inhibition zones observed for pharmaceutical composition "X" of the invention compared to those observed for Neosporin® or Silvadene® alone (Table 1 and FIG. 7) suggest that a patient can be treated effectively with lesser amounts of the full-strength active ingredients in Neosporin® and Silvadene® by using the exemplary pharmaceutical composition, "X". This is particularly advantageous because a patient can be treated with a much less concentrated dose of silver-containing antibiotic, thus minimizing potential side effects that are a concern for the use of silver in open wounds.

The coverage of pharmaceutical composition "X" of the invention against C. albicans is no different than Silvadene®, but is clearly superior to Neosporin® alone (Table 2). All formulations tested have good coverage against S. aureus, but pharmaceutical composition "X" and Neosporin® fared better than Silvadene® alone (Table 2). Even though the coverage of pharmaceutical composition "X" versus Silvadene® against MRSA was only a 1 mm difference, it is important to consider that the concentration of the silver sulfadiazine in pharmaceutical composition "X" is one-fourth (1/4) the strength of that contained in Silvadene®, further suggesting that one can get coverage against a broad range of microbes, including fungi and MRSA, using pharmaceutical composition "X" of the present invention compared to presently-used topical wound treatments. Furthermore, there were no differences observed between the ten (10) different MRSA strains when treated with pharmaceutical composition "X", indicating that this composition is effective against different genetic mutations of staphylococcus aureus MRSA strains observed in the clinic.

| **TABLE 2** | | | |
|---|---|---|---|
| ***in vitro* Zone Inhibition Significant Difference Comparisons** | | | |
| **Microbe** | **X** | **Neosporin®** | **Silvadene®** |
| **S. pyogenes (n = 6)** | **20 ± 0.5** | **15 ± 0.3** | **10 ± 0.3** |
| **C. albicans (n = 6)** | **11 ± 0.2** | **0** | **11 ± 0.2** |
| **S. aureus (n=6)** | **14 ± 0.4** | **14 ± 0.6** | **12 ± 0.2** |
| **MRSA (10 Strains, n = 30)** | **13 ± 0.1** | **0** | **12 ± 0.1** |
| Mean ± SEM, inhibition diameter (*mm*) | | | |
| ANOVA withTukey post hoc analysis | | | |
| Significant inhibition (*p<0.01*) | | | |
| The only non-significant differences within each microbe *vs.* X, N, and S are: | | | |
| C. albicans: X *vs.* S | | | |
| S. aureus: X *vs.* N | | | |

The case studies described in Example 1, and the culture and sensitivity *in vitro* studies in Example 2, demonstrate that a topical composition of the present invention exhibited surprising efficacy in mitigating the serious complications associated with acute and chronic wounds.

## Claims

1. Composition for use in the treatment of a skin condition, comprising a combination of agents, wherein the combination includes:
a) antimicrobial compounds, wherein the antimicrobial compounds include silver sulfadiazine, bacitracin, neomycin sulfate, and polymixin B sulfate;
b) one or more trace elements;
c) one or more tissue reconstruction elements; and
d) one or more analgesics,
and a pharmaceutically-acceptable carrier, and wherein the composition is a topical pharmaceutical composition.

2. The composition for use of Claim 1, wherein the combination is dispersed in an oil-in-water emulsion.

3. The composition for use of Claim 2, wherein the oil-in-water emulsion comprises a water-soluble carrier or a water-dispersible carrier.

4. The composition for use of Claim 1, 2 or 3, wherein the composition is formulated as an ointment, cream, gel, emulsion, poultice, lotion, foam, suspension, paste or tincture.

5. The composition for use of any one of Claims 1-4, (a) comprising about 0.1 to about 5% by weight of silver sulfadiazine, and/or (b) comprising about 0.1 to about 4000 Units per gram bacitracin.

6. The composition for use of any one of the preceding claims, wherein the one or more trace elements are selected from the group consisting of zinc, silver, selenium, copper, iron, magnesium, vitamin C, vitamin E and Coenzyme Q₁₀, or a combination thereof, optionally (a) wherein the one or more trace elements include zinc, and/or (b) comprising about 0.1 to about 40% by weight of zinc.

7. The composition for use of any one of the preceding claims, wherein the one or more tissue reconstruction elements are selected from the group consisting of vitamin E, Coenzyme Q₁₀, L-carnitine, choline, folic acid, magnesium, oleic acid, caprylic acid, linoleic acid, lauric acid, and taurine, or a combination thereof.

8. The composition for use of Claim 7, (a) wherein the one or more tissue reconstruction elements include vitamin E and Coenzyme Q₁₀, and/or (b) comprising about 0.05 to about 120,000 Units per gram vitamin E.

9. The composition for use of Claim 8, comprising about 1 to about 100 mg per gram Coenzyme Q₁₀.

10. The composition for use of any one of the preceding claims, wherein the one or more analgesics are derived from cocaine, optionally (a) wherein the one or more analgesics are selected from the group consisting of lidocaine, pramoxine, benzocaine, tetracaine, dibucaine, butamben, oxybuprocaine, proparacine, and proxymetacaine, or a combination thereof, and/or (b) wherein the one or more analgesics include lidocaine, and/or (c) comprising about 0.1 to about 25% by weight of lidocaine.

11. The composition for use of any one of the preceding claims, comprising zinc oxide, lidocaine, vitamin E and Coenzyme Q₁₀.

12. The composition for use of Claim 11, comprising:
a) about 0.25% silver sulfadiazine;
b) about 5% zinc oxide;
c) about 0.5% lidocaine;
d) about 400 Units per gram bacitracin;
e) about 3.5 mg per gram of neomycin;
f) about 5000 Units per gram polymixin B sulfate;
g) about 350 Units per gram Vitamin E; and
h) about 15 mg per gram Coenzyme Q₁₀.

13. The composition for use of any one of the preceding claims, wherein the composition is in a dosage form suitable for application to skin.

14. The composition for use of any one of the preceding claims, further comprising one or more inactive ingredients,
optionally wherein the one or more inactive ingredients are selected from the group consisting of petrolatum, a long chain fatty alcohol having a carbon atom content in the range C₁₆ - C₂₂, a non-ionic emulsifying agent, a humectant, a preservative and a cellulose gum derivative, or a combination thereof.

## Patentansprüche

1. Zusammensetzung zur Verwendung bei der Behandlung eines Hautzustands, die eine Kombination von Mitteln umfasst, wobei die Kombination umfasst:
a) antimikrobielle Verbindungen, wobei die antimikrobiellen Verbindungen Silbersulfadiazin, Bacitracin, Neomycinsulfat und Polymixin B-Sulfat umfassen;
b) ein oder mehrere Spurenelement(e);
c) ein oder mehrere Gewebewiederherstellungselement(e); und
d) ein Analgetikum oder mehrere Analgetika,
und einen pharmazeutisch verträglichen Träger, und wobei die Zusammensetzung eine topische pharmazeutische Zusammensetzung ist.

2. Zusammensetzung zur Verwendung nach Anspruch 1, bei der die Kombination in einer ÖI-in-Wasser-Emulsion dispergiert ist.

3. Zusammensetzung zur Verwendung nach Anspruch 2, bei der die ÖI-in-Wasser-Emulsion einen wasserlöslichen Träger oder einen wasserdispergierbaren Träger umfasst.

4. Zusammensetzung zur Verwendung nach Anspruch 1, 2 oder 3, wobei die Zusammensetzung als Salbe, Creme, Gel, Emulsion, Breipackung, Lotion, Schaum, Suspension, Paste oder Tinktur formuliert ist.

5. Zusammensetzung zur Verwendung nach einem der Ansprüche 1 bis 4, die (a) etwa 0,1 bis etwa 5 Gew.-% Silbersulfadiazin umfasst und/oder (b) etwa 0,1 bis etwa 4000 Einheiten pro Gramm Bacitracin umfasst.

6. Zusammensetzung zur Verwendung nach einem der vorhergehenden Ansprüche, bei der das eine oder die mehreren Spurenelement(e) aus der Gruppe, bestehend aus Zink, Silber, Selen, Kupfer, Eisen, Magnesium, Vitamin C, Vitamin E und Coenzym Q₁₀ oder einer Kombination davon, ausgewählt ist oder sind, gegebenenfalls (a) wobei das eine oder die mehreren Spurenelement(e) Zink umfasst oder umfassen und/oder (b) etwa 0,1 bis etwa 40 Gew.-% Zink umfasst oder umfassen.

7. Zusammensetzung zur Verwendung nach einem der vorhergehenden Ansprüche, bei der das eine oder die mehreren Gewebewiederherstellungselement(e) aus der Gruppe, bestehend aus Vitamin E, Coenzym Q₁₀, L-Carnitin, Cholin, Folsäure, Magnesium, Ölsäure, Caprylsäure, Linolsäure, Laurinsäure und Taurin oder einer Kombination davon, ausgewählt ist oder sind.

8. Zusammensetzung zur Verwendung nach Anspruch 7, (a) bei der das eine oder die mehreren Gewebewiederherstellungselement(e) Vitamin E und Coenzym Q₁₀ umfasst oder umfassen, und/oder (b) etwa 0,05 bis etwa 120000 Einheiten pro Gramm Vitamin E umfasst oder umfassen.

9. Zusammensetzung zur Verwendung nach Anspruch 8, die etwa 1 bis etwa 100 mg pro Gramm Coenzym Q₁₀ umfasst.

10. Zusammensetzung zur Verwendung nach einem der vorhergehenden Ansprüche, bei der das eine Analgetikum oder die mehreren Analgetika von Kokain abgeleitet ist oder sind, gegebenenfalls (a) wobei das eine Analgetikum oder die mehreren Analgetika aus der Gruppe, bestehend aus Lidocain, Pramoxin, Benzocain, Tetracain, Dibucain, Butamben, Oxybuprocain, Proparacin und Proxymetacain oder einer Kombination davon, ausgewählt ist oder sind, und/oder (b) wobei das eine Analgetikum oder die mehreren Analgetika Lidocain umfasst oder umfassen, und/oder (c) etwa 0,1 bis etwa 25 Gew.-% Lidocain umfasst oder umfassen.

11. Zusammensetzung zur Verwendung nach einem der vorhergehenden Ansprüche, die Zinkoxid, Lidocain, Vitamin E und Coenzym Q₁₀ umfasst.

12. Zusammensetzung zur Verwendung nach Anspruch 11, umfassend:
a) etwa 0,25 % Silbersulfadiazin;
b) etwa 5 % Zinkoxid;
c) etwa 0,5 % Lidocain;
d) etwa 400 Einheiten pro Gramm Bacitracin;
e) etwa 3,5 mg pro Gramm Neomycin;
f) etwa 5000 Einheiten pro Gramm Polymixin B-Sulfat;
g) etwa 350 Einheiten pro Gramm Vitamin E; und
h) etwa 15 mg pro Gramm Coenzym Q₁₀.

13. Zusammensetzung zur Verwendung nach einem der vorhergehenden Ansprüche, wobei die Zusammensetzung in einer Dosierungsform vorliegt, die für eine Anwendung auf die Haut geeignet ist.

14. Zusammensetzung zur Verwendung nach einem der vorhergehenden Ansprüche, die ferner einen oder mehrere inaktive(n) Bestandteil(e) umfasst, wobei gegebenenfalls der eine oder die mehreren inaktive(n) Bestandteil(e) aus der Gruppe, bestehend aus Vaseline, einem langkettigen Fettalkohol mit einem Kohlenstoffatomgehalt im Bereich von C₁₆ bis C₂₂, einem nicht-ionischen Emulgator, einem Feuchthaltemittel, einem Konservierungsmittel und einem Cellulosegummiderivat oder einer Kombination davon, ausgewählt ist oder sind.

## Revendications

1. Composition pour une utilisation dans le traitement d'une affection cutanée, comprenant une combinaison d'agents, la combinaison comprenant :
a) des composés antimicrobiens, les composés antimicrobiens comprenant de la sulfadiazine d'argent, de la bacitracine, du sulfate de néomycine et du sulfate de polymixine B ;
b) un ou plusieurs éléments sous forme de traces ;
c) un ou plusieurs éléments de reconstruction tissulaire ; et
d) un ou plusieurs analgésiques,
e) et un support pharmaceutiquement acceptable, et la composition étant une composition pharmaceutique topique.

2. Composition pour une utilisation selon la revendication 1, dans laquelle la combinaison est dispersée dans une émulsion huile-dans-eau.

3. Composition pour une utilisation selon la revendication 2, dans laquelle l'émulsion huile-dans-eau comprend un support soluble dans l'eau ou un support dispersible dans l'eau.

4. Composition pour une utilisation selon l'une des revendications 1, 2 ou 3, dans laquelle la composition est formulée sous la forme d'une pommade, d'une crème, d'un gel, d'une émulsion, d'un cataplasme, d'une lotion, d'une mousse, d'une suspension, d'une pâte ou d'une teinture.

5. Composition pour une utilisation selon l'une quelconque des revendications 1-4, (a) comprenant environ 0,1 à environ 5 % en poids de sulfadiazine d'argent, et/ou (b) comprenant environ 0,1 à environ 4 000 Unités par gramme de bacitracine.

6. Composition pour une utilisation selon l'une quelconque des revendications précédentes, dans laquelle le ou les éléments sous forme de traces sont choisis dans le groupe consistant en le zinc, l'argent, le sélénium, le cuivre, le fer, le magnésium, la vitamine C, la vitamine E et la Coenzyme Q₁₀, ou une combinaison de ceux-ci, facultativement (a) dans laquelle le ou les éléments sous forme de traces comprennent le zinc, et/ou (b) comprenant environ 0,1 à environ 40 % en poids de zinc.

7. Composition pour une utilisation selon l'une quelconque des revendications précédentes, dans laquelle le ou les éléments de reconstruction tissulaire sont choisis dans le groupe consistant en la vitamine E, la Coenzyme Q₁₀, la L-carnitine, la choline, l'acide folique, le magnésium, l'acide oléique, l'acide caprylique, l'acide linoléique, l'acide laurique et la taurine, ou une combinaison de ceux-ci.

8. Composition pour une utilisation selon la revendication 7, (a) dans laquelle le ou les éléments de reconstruction tissulaire comprennent la vitamine E et la Coenzyme Q₁₀, et/ou (b) comprenant environ 0,05 à environ 120 000 Unités par gramme de vitamine E.

9. Composition pour une utilisation selon la revendication 8, comprenant environ 1 à environ 100 mg par gramme de Coenzyme Q₁₀.

10. Composition pour une utilisation selon l'une quelconque des revendications précédentes, dans laquelle le ou les analgésiques sont issus de la cocaïne, facultativement (a) dans laquelle le ou les analgésiques sont choisis dans le groupe consistant en la lidocaïne, la pramoxine, la benzocaïne, la tétracaïne, la dibucaïne, le butambène, l'oxybuprocaïne, la proparacine et la proxymétacaïne, ou une combinaison de ceux-ci,
et/ou (b) dans laquelle le ou les analgésiques comprennent la lidocaïne,
et/ou (c) comprenant environ 0,1 à environ 25 % en poids de lidocaïne.

11. Composition pour une utilisation selon l'une quelconque des revendications précédentes, comprenant l'oxyde de zinc, la lidocaïne, la vitamine E et la Coenzyme Q₁₀.

12. Composition pour une utilisation selon la revendication 11, comprenant :
a) environ 0,25 % de sulfadiazine d'argent ;
b) environ 5 % d'oxyde de zinc ;
c) environ 0,5 % de lidocaïne ;
d) environ 400 Unités par gramme de bacitracine ;
e) environ 3,5 mg par gramme de néomycine ;
f) environ 5 000 Unités par gramme de sulfate de polymixine B ;
g) environ 350 Unités par gramme de Vitamine E ; et
h) environ 15 mg par gramme de Coenzyme Q₁₀.

13. Composition pour une utilisation selon l'une quelconque des revendications précédentes, dans laquelle la composition est dans une forme posologique appropriée pour l'application sur la peau.

14. Composition pour une utilisation selon l'une quelconque des revendications précédentes, comprenant en outre un ou plusieurs ingrédients inactifs, facultativement dans laquelle le ou les ingrédients inactifs sont choisis dans le groupe consistant en la vaseline, un alcool gras à longue chaîne ayant une teneur en atomes de carbone dans la plage de C₁₆-C₂₂, un agent émulsifiant non ionique, un humectant, un conservateur et un dérivé de gomme de cellulose, ou une combinaison de ceux-ci.
